(19) Europäisches Patentamt
European Patent Office
Office européen des brevets

(11) **EP 2 836 189 B1**

(12) **FASCICULE DE BREVET EUROPEEN**

(45) Date de publication et mention de la délivrance du brevet:
**09.08.2017 Bulletin 2017/32**

(21) Numéro de dépôt: **13720469.9**

(22) Date de dépôt: **10.04.2013**

(51) Int Cl.:
*A61K 8/37* (2006.01)   *A61K 8/49* (2006.01)
*A61Q 19/08* (2006.01)

(86) Numéro de dépôt international:
**PCT/FR2013/050780**

(87) Numéro de publication internationale:
**WO 2013/153330 (17.10.2013 Gazette 2013/42)**

(54) **COMPOSITION STABLE COMPRENANT UN COMPOSÉ ANTI-ÂGE ET UN SOLVANT**

STABILE ZUSAMMENSETZUNG MIT EINER ALTERUNGSHEMMUNGSVERBINDUNG UND EINEM LÖSUNGSMITTEL

STABLE COMPOSITION COMPRISING AN ANTI-AGEING COMPOUND AND A SOLVENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.04.2012 FR 1253356**

(43) Date de publication de la demande:
**18.02.2015 Bulletin 2015/08**

(73) Titulaire: **Chanel Parfums Beauté**
**92200 Neuilly-sur-Seine (FR)**

(72) Inventeur: **FOURNIER, Odile**
**F-93694 Pantin Cedex (FR)**

(74) Mandataire: **Cabinet Plasseraud**
**66 rue de la Chaussée d'Antin**
**75440 Paris Cedex 09 (FR)**

(56) Documents cités:
**WO-A1-2009/129627    WO-A2-2004/103265**
**US-A1- 2008 139 507    US-A1- 2009 215 881**
**US-A1- 2011 251 242**

• **"HANSEN SOLUBILITY PARAMETERS OF SOLVENTS", INDUSTRIAL SOLVENTS HANDBOOK, XX, XX, 1 janvier 1996 (1996-01-01), pages 35-56, XP000944977,**

Remarques:
Le dossier contient des informations techniques présentées postérieurement au dépôt de la demande et ne figurant pas dans le présent fascicule.

**Description**

La présente invention concerne une composition cosmétique stable, et de préférence sans odeur désagréable, comprenant le diacétylresvératryl thioctate, un solvant et optionnellement un absorbeur d'odeur. Cette composition est utile pour prévenir et/ou lutter contre le vieillissement cutané.

L'utilisation du composé diacétylresvératryl thioctate a été divulguée dans la demande de brevet WO 2006/134282.

Néanmoins la Demanderesse a remarqué que ce composé pose un problème de stabilité lors de sa formulation, notamment s'il est solubilisé dans un solvant lipophile classique de type octyldodécanol. Or, il serait souhaitable de pouvoir disposer de compositions cosmétiques stables comprenant le diacétylresvératryl thioctate.

La Demanderesse a maintenant découvert que ce problème pouvait être résolu en utilisant un solvant spécifique qui a une valeur de $\delta_h$ inférieure ou égale à 8 et une valeur de $\delta_d$ supérieure ou égale à 16,9 à température ambiante.

La présente invention a donc pour objet une composition cosmétique de soin comprenant:

- le diacétylresvératryl thioctate et
- un solvant ;

ledit solvant ayant une valeur de $\delta_h$ inférieure ou égale à 8 et une valeur de $\delta_d$ supérieure ou égale à 16,9 à température ambiante.

Un tel solvant est de préférence choisi parmi : trioctyl trimellitate, isosorbide de diméthyle, éthylhexyle méthoxycrylène, octocrylène, benzoate de phényle et leurs mélanges.

Dans un mode de réalisation particulier, le solvant est choisi parmi : trioctyl trimellitate, isosorbide de diméthyle, éthylhexyle méthoxycrylène, octocrylène, méthyle trimellitate, benzoate de phényle et leurs mélanges.

L'invention a également pour objet l'utilisation d'une composition selon l'invention pour prévenir et/ou lutter contre le vieillissement de la peau.

Le diacétylresvératryl thioctate peut être préparé comme indiqué dans la demande de brevet WO 2006/134282.

La Demanderesse a en effet découvert que le diacétylresvératryl thioctate a un problème de stabilité : quant il est solubilisé dans un solvant classique de type octyldodécanol, il recristallise.

Or il est essentiel de pouvoir disposer de compositions dans lesquelles le diacétylresvératryl thioctate est stabilisé.

Par « **stabilisation** », on entend une absence de cristallisation du diacétylresvératryl thioctate après 10 jours à 4°C et 6 mois à $T_a$.

Par « $T_a$ », on entend la température ambiante.

Par « **température ambiante** », on entend une température d'environ 25 °C.

La caractéristique d'un solvant est décrite par cinq paramètres de solution : quatre paramètres de solubilité d'Hansen ($\delta_d$, $\delta_p$, $\delta_h$ et $\delta_T$) et Log ($K_{OW}$).

Les paramètres de solubilité d'Hansen ($\delta_d$, $\delta_p$, $\delta_h$ et $\delta_T$) permettent de prédire si une substance sera solubilisée dans une autre.

$\delta_d$ est une contribution due aux forces de dispersion, $\delta_p$ est une contribution due aux forces polaires, $\delta_h$ est une contribution de lien hydrogène. Ces paramètres sont définis comme suit :

$$\delta_d = \left(\frac{\Delta E_d}{V}\right)^{1/2}$$

$$\delta_p = \left(\frac{\Delta E_p}{V}\right)^{1/2}$$

$$\delta_h = \left(\frac{\Delta E_h}{V}\right)^{1/2}$$

Ici, $\Delta E$ correspond à l'énergie de vaporisation et V le volume molaire du liquide.

$\delta_T$ est le paramètre de solubilité dû à la contribution de chacun de ces paramètres :

$$\delta_T = \left(\delta_d^2 + \delta_p^2 + \delta_h^2\right)^{1/2}$$

Log ($K_{OW}$) est une mesure de la solubilité différentielle d'un composé chimique dans deux solvants (octanol et eau) et correspond au coefficient de partage octanol/eau.

La Demanderesse a maintenant découvert que ce problème pouvait être résolu en solubilisant le diacétylresvératryl thioctate dans un solvant particulier, qui a une valeur de $\delta_h$ inférieure ou égale à 8 et une valeur de $\delta_d$ supérieure ou égale à 16,9, à température ambiante.

Des valeurs de $\delta_h$ et $\delta_d$ dans ces gammes permettent d'assurer que ledit solvant facilite la solubilisation stable du diacétylresvératryl thioctate.

A la connaissance de la Demanderesse, il n'a encore jamais été suggéré d'utiliser un solvant ayant une valeur de $\delta_h$ inférieure ou égale à 8 et une valeur de $\delta_d$ supérieure ou égale à 16,9 à température ambiante, dans le but de stabiliser cet actif.

Dans un mode de réalisation particulier, le solvant de la composition cosmétique selon la présente invention a une valeur de $\delta_p$ comprise entre 4,7 et 7 à température ambiante.

Une valeur de $\delta_p$ dans cette gamme permet d'assurer que ledit solvant facilite la solubilisation stable de l'actif.

Dans un mode de réalisation particulier, le solvant de la composition cosmétique selon la présente invention a une valeur de $\delta_T$ comprise entre 18 et 22 à température ambiante.

Une valeur de $\delta_T$ dans cette gamme permet d'assurer que ledit solvant facilite la solubilisation stable de l'actif.

Dans un mode de réalisation particulier, le solvant de la composition cosmétique selon la présente invention a une valeur de log($K_{ow}$) comprise entre 6 et 7,5 à température ambiante.

Une valeur de log($K_{ow}$) dans cette gamme permet d'assurer que ledit solvant facilite la solubilisation stable de l'actif.

Dans un mode de réalisation particulier, le solvant de la composition cosmétique selon la présente invention est choisi parmi : trioctyl trimellitate, isosorbide de diméthyle, éthylhexyle méthoxycrylène, octocrylène, benzoate de phényle et leurs mélanges.

Dans un mode de réalisation particulier, le solvant est choisi parmi : trioctyl trimellitate, isosorbide de diméthyle, éthylhexyle méthoxycrylène, octocrylène, méthyle trimellitate, benzoate de phényle et leurs mélanges.

Dans un mode de réalisation préférée, le solvant est l'octocrylène.

Le diacétylresvératryl thioctate peut être présent, dans la composition de la présente invention, en une proportion allant de 0,001 % à 15 % en poids, et de préférence de 0,01 % à 10 % en poids par rapport au poids total de la composition. Le solvant peut être présent, dans la composition de la présente invention, en une proportion allant de 0,1 % à 50 % en poids, et de préférence de 1 % à 25 % en poids par rapport au poids total de la composition.

Bien que le diacétylresvératryl thioctate soit stabilisé physiquement par un solvant selon l'invention, c'est-à-dire qu'il est solubilisé par ledit solvant sans cristallisation, la composition peut poser un problème de stabilité olfactive. En effet, le développement d'une odeur difficilement acceptable pour un produit cosmétique a été observé.

La Demanderesse a maintenant découvert que ce problème pouvait être résolu en ajoutant, à la composition décrite ci-dessus, un capteur d'odeur choisi parmi: magnésium aluminium silicate hydraté, oxyde de zinc, kaolin, $Cu_2CO_3(OH)_2$ (ou malachite), sel de cuivre et de L-pyrrolidone carboxylate et leurs mélanges.

Par « **capteur d'odeur** » ou « **absorbeur d'odeur** », on entend une molécule qui a une affinité avec le soufre et qui permet de neutraliser l'odeur difficilement acceptable.

Ainsi dans un mode de réalisation particulier, la composition cosmétique de la présente invention comprend en outre un absorbeur d'odeur choisi parmi : magnésium aluminium silicate hydraté, oxyde de zinc, kaolin, $Cu_2CO_3(OH)_2$ (ou malachite), sel de cuivre et de L-pyrrolidone carboxylate et leurs mélanges.

De préférence, un absorbeur d'odeur est choisi parmi : kaolin, sel de cuivre et de L-pyrrolidone carboxylate et leurs mélanges.

L'absorbeur d'odeur peut être présent, dans la composition de la présente invention, en une proportion allant de 0,0006 % à 6 % en poids, et de préférence de 0,006 % à 0,6 % en poids par rapport au poids total de la composition.

Outre le diacétylresvératryl thioctate, le solvant spécifique et de préférence un absorbeur d'odeur précédemment décrit, la composition cosmétique selon l'invention peut également comprendre au moins un additif usuel dans le domaine cosmétique ou de la pharmacie, tel que par exemple un composé choisi parmi un agent gélifiant et/ou épaississant, un agent tensioactif ou co-tensioactif, un corps gras liquide ou une huile, une cire, un élastomère de silicone, un filtre solaire, un colorant, un agent matifiant ou une charge, un pigment, un agent tenseur, un conservateur, un séquestrant, un parfum et leurs mélanges.

Notamment, selon un mode de réalisation préféré, la composition cosmétique selon l'invention peut comprendre, de manière non limitative, un ou plusieurs des additifs suivants :

- un ou plusieurs agent(s) gélifiant(s) et/ou épaississant(s) de la phase aqueuse, choisi par exemple parmi les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique tels que les ammonium acryloyldiméthyltaurate/VP copolymer et ammonium acryloyldiméthyl-taurate/beheneth-25 méthacrylate copolymer, notamment ceux vendus sous les dénominations Aristoflex® AVC et HMB de Clariant, ou encore les acrylates/C10-30 Alkyl Acrylate Crosspolymer vendu sous la dénomination commerciale PEMULEN® TR-1 ou TR-2, Carbopol® 1382, Carbopol® Ultrez 20 par la société Novéon, les dérivés cellulosiques, les gommes d'origine végétale (acacia ou arabique, agar, guar, caroube, alginates, carraghénanes, pectine) ou d'origine microbienne (xanthane, pullulane), les argiles (laponite). Ledit agent gélifiant et/ou épaississant peut être présent dans la composition en une teneur de l'ordre de 0,01 à 5% en poids, par rapport au poids total de la composition ;

- un ou plusieurs agent(s) tensioactif(s), de préférence émulsionnants, qu'ils soient non ioniques, anioniques, cationiques ou amphotères, et en particulier les esters d'acides gras et de polyols tel que les esters d'acides gras et de glycérol oxyalkylénés (plus particulièrement polyoxyéthylénés), les esters d'acides gras et de sorbitan oxyalkylénés, les esters d'acides gras oxyalkylénés (oxyéthylénés et/ou oxypropylénés) comme le mélange PEG-100 Stearate/Glyceryl Stearate commercialisé par exemple par la société Croda sous la dénomination Arlacel® 165 et les esters d'acides gras et de sucrose comme le stéarate de sucrose; les éthers d'alcool gras et de sucre, notamment les alkylpolyglucosides (APG) tels que le décylglucoside et le laurylglucoside, le cétostéarylglucoside éventuellement en mélange avec l'alcool cétostéarylique, commercialisé par exemple sous la dénomination Montanov® 68 par la société Seppic, ainsi que l'arachidyl glucoside, par exemple sous la forme du mélange d'alcools arachidique et béhénique et d'arachidylglucoside commercialisé sous la dénomination Montanov® 202 par la société Seppic ; les éthers d'alcools gras et de polyéthylèneglycol; les polysiloxanes modifiés polyéthers; la bétaïne et ses dérivés; les polyquaterniums; les sels de sulfate d'alcools gras éthoxylés; les sulfosuccinates; les sarcosinates; les alkyl- et dialkylphosphates et leurs sels; et les savons d'acides gras. Ledit agent tensioactif peut être présent dans la composition dans une teneur de l'ordre de 0,1 à 8%, de préférence 0,5 à 3% en poids, par rapport au poids total de la composition ;

- un ou plusieurs co-tensioactif(s) tels que les alcools gras linéaires à longue chaîne carbonée (C14-C20) et en particulier les alcools cétylique et stéarylique, ledit agent tensioactif étant présent dans la composition à raison de 0,1 à 5%, de préférence 0,5 à 2% en poids, par rapport au poids total de la composition ;

- un ou plusieurs corps gras liquide(s) à température ambiante, communément dénommé(s) huiles(s), volatile (s) ou non volatile(s), hydrocarboné(s), siliconée(s), linéaire(s), cyclique(s) ou ramifiée(s), par exemple, les huiles de silicone telles que les polydiméthylsiloxanes (diméthicones), les polyalkylcyclosiloxanes (cyclométhicones) et les polyalkylphénylsiloxanes (phenyldiméthicones); les huiles synthétiques telles que les huiles fluorées, les alkyl benzoates et les hydrocarbures ramifiés tels que le polyisobutylène, l'isododécane; les huiles minérales (paraffine); végétales (huile d'amande douce, de macadamia, de pépin de cassis, de jojoba ou encore de camelina sativa telle que l'huile vendue sous la dénomination commerciale Lipex® Oméga 3/6 par la société Unipex) ; les alcools gras, les amides grasses, les acides ou les esters gras comme le benzoate d'alcools en C12-C15 vendu sous la dénomination commerciale Finsolv® TN par la société Innospec, les triglycérides dont ceux des acides caprique/caprylique, le dicaprylyl carbonate vendu sous la dénomination Cetiol® CC par la société Cognis ; de préférence à raison de 0,1 à environ 10%, de préférence, de 0,5 à 5% en poids, par rapport au poids total de la composition ;

- une ou plusieurs cires (composé solide ou substantiellement solide à température ambiante), et dont le point de fusion est généralement supérieur à 35°C, telles que l'ozokérite, la cire de polyéthylène, la cire d'abeille ou la cire de carnauba, de préférence à raison de 0,01 à environ 5%, de préférence 0,5 à 5% en poids, par rapport au poids total de la composition ;

- un ou plusieurs élastomère(s) de silicone obtenus notamment par réaction, en présence d'un catalyseur, d'un polysiloxane ayant au moins un groupe réactif (hydrogène ou vinyle, notamment) et portant au moins un groupe alkyle (notamment méthyle) ou phényle, terminal et/ou latéral, avec un organosilicone tel qu'un organohydrogénopolysiloxane, de préférence à raison de 0,1 à environ 20%, de préférence 0,25 à 15% en poids, par rapport au poids total de la composition ;

- un ou plusieurs filtre(s) solaire(s), notamment les filtres organiques, tels que les dérivés de dibenzoylméthane (dont le butyl méthoxydibenzoylméthane vendu en particulier par DSM sous le nom commercial Parsol® 1789), les dérivés d'acide cinnamique (dont l'éthylhexyle méthoxycinnamate vendu en particulier par DSM sous le nom commercial Parsol® MCX), les salicylates, les acides para-aminobenzoïques, les β-β'-diphénylacrylates, les benzophénones, les dérivés de benzylidène camphre, les phénylbenzimidazoles, les triazines, les phénylbenzotriazoles et les dérivés anthraniliques ; ou les filtres inorganiques, à base d'oxydes minéraux sous forme de pigments ou de nanopigments, enrobés ou non, et en particulier à base de dioxyde de titane ou d'oxyde de zinc ; de préférence à raison de 0,1 à environ 30%, mieux, de 0,5 à 20% en poids, par rapport au poids total de la composition ;

- un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0,1 à environ 2% en poids, par rapport au poids total de la composition ;

- une ou plusieurs charges, en particulier des agents matifiants ou des charges à effet flouteur, et en particulier les poudres à effet soft-focus.

Par charge, il faut comprendre des particules incolores ou blanches, minérales ou de synthèse, lamellaires ou non lamellaires, adaptées à donner du corps ou de la rigidité à la composition et/ou de la douceur, de la matité et de l'uniformité immédiate à l'application. Ces charges peuvent notamment modifier voire masquer les rides par un effet de camouflage, ou un effet de floutage.

Les agents matifiants peuvent être choisis parmi les polymères matifiants (en solution, en dispersion ou sous forme de particules) et les particules inorganiques qui réduisent la brillance de la peau et unifient le teint. L'agent matifiant pourra notamment être choisi parmi un amidon, le talc, des microbilles de cellulose, des fibres végétales, des fibres synthétiques, en particulier de polyamides (les poudres de Nylon® telles que Nylon-12 (Orgasol® commercialisé par la société Atochem), des microsphères de copolymères acryliques notamment de poly(méth)acrylate de méthyle (particules PMMA ou les particules Micropearl® M310 vendue par la société Seppic), les poudres de silice, les poudres de résine de silicone, les poudres de polymères acryliques, les poudres de polyéthylène, les organopolysiloxanes réticulés élastomères (commercialisés notamment sous les dénominations KSG® par la société Shin-Etsu, sous les dénominations Trefil®, BY29® ou EPSX® par la société Dow Corning ou sous les dénominations Gransil® par la société Grant Industries), les poudres composites de talc/dioxyde de titane/alumine/silice, les poudres de silicates, et leurs mélanges.

La charge à effet « soft focus » peut donner de la transparence au teint et un effet flou. De préférence, les charges « soft focus » ont une taille moyenne de particules inférieure ou égale à 30 microns, plus préférentiellement inférieure ou égale à 15 microns. Ces charges « soft focus » peuvent être de toutes formes et en particulier être sphériques ou non sphériques. Elles peuvent être choisies parmi les poudres de silice et silicates, notamment d'alumine, les poudres de type polyméthyl méthacrylate (PMMA ou Micropearl® M310), le talc, les composites silice/TiO2 ou silice/oxyde de zinc, les poudres de polyéthylène, les poudres d'amidon, les poudres de polyamides, les poudres de copolymères styrène/acrylique, les élastomères de silicone, et leurs mélanges.

De préférence ces agents matifiants ou charges à effet soft-focus sont utilisés à raison de 0,1 à environ 10% en poids, par rapport au poids total de la composition, de préférence à raison de 0,1 à environ 7% en poids.

Une ou plusieurs pigments blancs ou colorés, nacrés ou non, minéraux et/ou organiques, enrobés ou non, insolubles dans le milieu, destinées à colorer et/ou opacifier la composition. Ils peuvent être de taille usuelle ou nanométrique. On peut citer, parmi les pigments minéraux, le dioxyde de titane, éventuellement traité en surface, les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium. Les pigments nacrés ou nacres sont des particules irisées qui réfléchissent la lumière. Ces pigments nacrés peuvent être choisis parmi les pigments nacrés blancs tels que le mica recouvert de titane, ou d'oxychlorure de bismuth, les pigments nacrés colorés tels que le mica titane avec des oxydes de fer. Les pigments peuvent avoir subi un traitement de surface. De préférence, ces pigments sont utilisés à raison de 0,1 à environ 10% en poids, par rapport au poids total de la composition, de préférence à raison de 0,1 à environ 5% en poids.

- un ou plusieurs agents tenseurs. Par agent tenseur, il faut comprendre un composé adapté à tendre la peau et, par cet effet de tension, lisser la peau et y faire diminuer voire disparaître de façon immédiate les rides et les ridules. Comme agents tenseurs, on peut citer les polymères d'origine naturelle ; les silicates mixtes ; les particules colloïdales de charges inorganiques ; les polymères synthétiques ; et leurs mélanges. On peut citer notamment : les polymères d'origine végétale ou microbienne, les polymères issus des phanères, les protéines d'oeuf et les latex d'origine naturelle. Ces polymères sont de préférence hydrophiles. Comme polymères d'origine végétale, on peut citer en particulier les protéines et hydrolysats de protéines, et plus particulièrement les extraits de céréales, de légumineuses et d'oléagineuses, tels que les extraits de maïs, de seigle, de froment, de sarrasin, de sésame, d'épeautre, de pois, de tapioca, de fève, de lentille, de soja et de lupin. D'autres agents tenseurs pouvant être mis en oeuvre selon l'invention sont les polysaccharides d'origine naturelle, notamment l'amidon issu notamment de riz, de maïs, de tapioca, de pomme de terre, de manioc, de pois ; les carraghénanes, gommes d'acacia (gomme arabique), alginates, agars, gellanes, gommes xanthanes, polymères cellulosiques et pectines, avantageusement en dispersion aqueuse de microparticules de gel, les dérivés cellulosiques, et leurs mélanges. Les polymères synthétiques se présentent généralement sous forme d'un latex ou d'un pseudolatex et peuvent être de type polycondensat ou obtenus par polymérisation radicalaire. On peut citer notamment les dispersions de polyester/polyuréthane et de polyéther/polyuréthane. De préférence, l'agent tenseur est un copolymère de PVP/dimethiconylacrylate et de polyuréthane hydrophile (Aquamere® S-2011® de la société Hydromer). Plus préférentiellement, l'agent tenseur est un extrait de tubercule de *Manihot esculenta* (vendu sous la dénomination INSTENSYL par Silab);

- un ou plusieurs conservateur(s) ;

- les séquestrants tels que les sels d'EDTA;

- les parfums;

- et leurs mélanges.

Des exemples de tels additifs sont cités notamment dans le Dictionnaire CTFA (International Cosmetic Ingrédient Dictionary and Handbook publié par The Cosmetic, Toiletry and Fragrance Association, 11ème Edition, 2006) qui décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

L'homme du métier est en mesure de choisir, parmi l'ensemble de ces éventuels additifs, aussi bien la composition que la quantité de ceux qui seront ajoutés à la composition, de telle sorte que celle-ci conserve l'ensemble de ses propriétés.

En outre, la composition selon la présente invention peut éventuellement contenir divers agents actifs qui peuvent être choisis dans le groupe constitué des vitamines, des antioxydants, des agents hydratants, des agents anti-pollution, des agents kératolytiques, des astringents, des anti-inflammatoires, des agents blanchissants et des agents favorisant la microcirculation.

Des exemples de vitamines incluent les vitamines A, B1, B2, B6, C et E et leurs dérivés, l'acide pantothénique et ses dérivés et la biotine.

Des exemples d'antioxydants incluent l'acide ascorbique et ses dérivés tels que le palmitate d'ascorbyle, le tétraisopalmitate d'ascorbyle, l'ascorbyl glucoside, le magnésium ascorbyl phosphate, le sodium ascorbyl phosphate et le sorbate d'ascorbyle; le tocophérol et ses dérivés, tels que l'acétate de tocophérol, le sorbate de tocophérol et d'autres esters de tocophérol; le BHT et BHA; les esters de l'acide gallique, l'acide phosphorique, l'acide citrique, l'acide maléique, l'acide malonique, l'acide succinique, l'acide fumarique, la céphaline, l'hexamétaphosphate, l'acide phytique, et les extraits de plantes, par exemple de racines de Zingiber Officinale (Gingembre) tel que le Blue Malagasy Ginger commercialisé par la société BIOLANDES, de Chondrus crispus, Rhodiola, Thermus thermophilus, la feuille de maté, le bois de chêne, l'écorce de Rapet Kayu, les feuilles de Sakura et les feuilles d'ylang ylang.

Des exemples d'agents hydratants incluent le polyéthylène glycol, le propylène glycol, le dipropylène glycol, la glycérine, le butylène glycol, le xylitol, le sorbitol, le maltitol, les mucopolysaccharides, tels que l'acide chondroïtine sulfurique, l'acide hyaluronique de haut ou de bas poids moléculaire ou encore l'acide hyaluronique potentialisé par un dérivé de silanol tel que l'actif Epidermosil® commercialisé par la société Exymol, et l'acide mucoitinsulfurique; l'acide caronique; les sels biliaires, une composante principale du FHN (facteur d'hydratation naturelle) comme un sel de l'acide pyrrolidone carboxylique et un sel d'acide lactique, un analogue d'acide aminé tel que l'urée, la cystéine et la sérine; un collagène soluble à chaîne courte, les PPG diglycérine, les homo- et copolymères de 2-méthacryloyloxyéthylphosphorylcholine comme le Lipidure HM et le Lipidure PBM de NOF; l'allantoïne; des dérivés de glycérine tels que le PEG / PPG / polybutylène Glycol-8/5/3 Glycérine de NOF vendu sous la dénomination commerciale Wilbride®S753 ou encore le glyceryl-polymethacrylate de Sederma vendu sous la dénomination commerciale Lubragel®MS; la triméthylglycine vendu sous la dénomination commerciale Aminocoat® par la société Ashahi Kasei Chemicals et divers extraits de plantes tels que des extraits de Castanea sativa, des protéines de noisette hydrolysées, les polysaccharides de Tuberosa Polyanthes, l'huile de noyau d'Argania spinosa et les extraits de nacre contenant un conchyoline qui sont vendus notamment par la compagnie Maruzen (Japon) sous le nom commercial Pearl Extract®.

D'autres exemples d'agents hydratants incluent les composés stimulant l'expression de la matriptase MT/SP1, tel qu'un extrait de pulpe de caroube, ainsi que les agents stimulant l'expression de FN3K; les agents augmentant la prolifération ou la différenciation des kératinocytes tels que les extraits de *Thermus thermophilus* ou de fleur de *Camellia Japonica Alba Plena* ou de coques de fèves de *Theobroma cacao,* les extraits hydrosolubles de maïs, les extraits peptidiques de *Voandzeia subterranea* et le niacinamide ; les lipides épidermiques et les agents augmentant la synthèse de lipides épidermiques, soit directement, soit en stimulant certaines β-glucosidases qui modulent la déglycosylation de précurseurs lipidiques comme le glucosylcéramide en céramides, tels que les phospholipides, les céramides, les hydrolysats de protéine de lupin.

Des exemples d'agents anti-pollution incluent l'extrait de graines de Moringa pterygosperma (par exemple le Purisoft® de LSN); l'extrait de beurre de karité (par exemple Detoxyl® de Silab), un mélange d'extrait de lierre, d'acide phytique, d'extrait de graine de tournesol (par exemple l'Osmopur® de Sederma).

Des exemples d'agents kératolytiques incluent les $\alpha$-hydroxyacides (par exemple les acides glycolique, lactique, citrique, malique, mandélique, ou tartrique) et les $\beta$-hydroxyacides (par exemple l'acide salicylique), et leurs esters, tels que les C12-13 alkyl lactates, et les extraits de plantes contenant ces hydroxyacides, tels que des extraits d'Hibiscus sabdriffa.

Des exemples d'agents anti-inflammatoires incluent le bisabolol, l'allantoïne, l'acide tranexamique, l'oxyde de zinc, l'oxyde de soufre et ses dérivés, le sulfate de chondroïtine, l'acide glycyrrhizinique et ses dérivés tels que les glycyrrhizinates.

Des exemples d'astringents incluent les extraits d'hamamélis.

Des exemples d'agents blanchissants incluent l'arbutine et ses dérivés, l'acide férulique (tel que le Cytovector® : eau, glycol, lécithine, acide férulique, hydroxyéthylcellulose, commercialisé par BASF) et ses dérivés, l'acide kojique, le résorcinol, l'acide ellagique, le leucodopachrome et ses dérivés, la vitamine B3, l'acide linoléique et ses dérivés, les céramides et leurs homologues, un peptide tel que décrit dans la demande de brevet WO2009010356, un bioprécurseur tel que décrit dans la demande de brevet WO2006134282 ou un sel de tranexamate tel que le sel de chlorhydrate de tranexamate cétylique, un extrait de réglisse (extrait de Glycyrrhiza glabra), qui est vendu notamment par la société Maruzen sous le nom commercial Licorice extract®, un agent blanchissant ayant également un effet antioxydant, comme les composés de vitamine C, y compris les sels d'ascorbate, les esters ascorbyle d'acides gras ou d'acide sorbique, et d'autres dérivés de l'acide ascorbique, par exemple, les phosphates d'ascorbyle, tels que le magnésium ascorbyl phosphate et le sodium ascorbyl phosphate, ou les esters de saccharide d'acide ascorbique, qui incluent, par exemple, l'ascorbyle-2-glucoside, le L-ascorbate de 2-0-alpha-D-glucopyranosyle, ou le L-ascorbate de 6-0-bêta-D-galactopyranosyle. Un agent actif de ce type est vendu en particulier par la société DKSH sous le nom commercial Ascorbyl glucoside ®.

Des exemples d'agents favorisant la microcirculation incluent un extrait de lupin (tel que l'Eclaline® de Silab), de ruscus, de marron d'inde, de lierre, de ginseng ou de mélilot, la caféine, le nicotinate et ses dérivés, un extrait d'algue de Corallina officinalis tel que celui commercialisé par CODIF ; et leurs mélanges. Ces agents actifs sur la microcirculation cutanée peuvent être utilisés pour éviter le ternissement du teint et/ou améliorer l'homogénéisation et l'éclat du teint.

La composition utilisée selon l'invention peut en outre comprendre en plus du polypeptide selon l'invention, au moins un actif choisi parmi : les agents stimulant l'expression de la tensine 1 tel qu'un extrait d'élémi; les agents stimulant l'expression de la FN3K et/ou de la FN3K RP tel qu'un extrait de *Butea frondosa ;* les agents stimulant l'expression de CERT ou d'ARNT2; les agents stimulant la production de facteurs de croissance; les agents anti-glycation ou déglycants; les agents augmentant la synthèse de collagène ou prévenant sa dégradation (agents anti-collagénases, notamment inhibiteurs de métalloprotéinases matricielles) en particulier les agents augmentant la synthèse de collagène IV et/ou de hyaluronane et/ou de fibronectine, tel qu'au moins un oligopeptide acylé, notamment celui commercialisé par la société SEDERMA sous la dénomination commerciale Matrixyl® 3000; les agents augmentant la synthèse d'élastine ou prévenant sa dégradation (agents anti-élastases); les agents augmentant la synthèse de glycosaminoglycanes ou de protéoglycanes ou prévenant leur dégradation (agents anti-protéoglycanases) tel que l'actif Epidermosil® (acide hyaluronique associé au méthylsilanetriol) commercialisé par la société Exsymol; les agents stimulant la synthèse d'intégrines par les fibroblastes ; les agents augmentant la prolifération des fibloblastes; les agents facilitant l'absorption percutanée tels que les alcools, les alcools gras et les acides gras et leurs dérivés ester ou éther, les pyrrolidones, les 4-alkyl-oxazolidin-2-ones telle que la 4-decyloxazolidin-2-one ; les terpènes, les huiles essentielles et les $\alpha$-hydroxyacides; et leurs mélanges, sans que cette liste ne soit limitative.

L'invention sera maintenant illustrée par les exemples non limitatifs suivants.

## EXEMPLES

### Exemple 1-a) : Choix de solvant

Des solvants candidats ont été choisis en utilisant le logiciel HSPiP (Version : 3.1.14).

Les solvants candidats ainsi choisis sont ensuite soumis au test de stabilisation. Ce test consiste à comparer la solubilité initiale ($t_0$) et celle d'un jour après (1J) à température ambiante ($T_a$).

### Résultats

Le tableau de la page suivante présente les valeurs des paramètres de solubilisation de chaque solvant candidat ($\delta_h$, $\delta_d$, $\delta_p$, $\delta_T$ et log($K_{ow}$) à température ambiante) et les résultats des tests de stabilisation du diacétylresvératryl thioctate.

| | Paramètres de solubilisation | | | | | solubilité | | Stabilité |
|---|---|---|---|---|---|---|---|---|
| | $\delta_h$ | $\delta_d$ | $\delta_p$ | $\delta_T$ | Log ($K_{ow}$) | $t_0/T_a$ | 1J/$T_a$ | |
| Trioctyl trimellitate | 2,1 | 16,9 | 4,7 | 18 | 9,71 | Partiel -lement soluble | limpide | oui |
| Isosorbide de diméthyle | 7,6 | 17,5 | 7,4 | 20 | -0,43 | Partiel -lement soluble | limpide | oui |
| Ethylhexyle méthoxycrylène | 6,4 | 18,1 | 6,8 | 20,2 | 6, 99 | Partiel -lement soluble | limpide | oui |
| Octocrylène | 5,6 | 18,2 | 6,6 | 19,5 | 6, 96 | Partiel -lement soluble | limpide | oui |
| Benzoate de phényle | 4,5 | 19,5 | 5,8 | 21 | 4,22 | Partiel -lement soluble | limpide | oui |
| Octyldodécanol | 9 | 16,1 | 3,8 | 19,1 | 8,84 | Insoluble | précipité | non |
| PPG-15 éther stéarylique | 8,2 | 16,4 | 5,8 | 19,5 | 8,35 | Insoluble | précipité | non |

Parmi les solvants testés, le diacétylresvératryl thioctate est partiellement soluble à $t_0$ et à $T_a$ dans le trioctyl trimellitate, l'isosorbide de diméthyle, l'éthylhexyle méthoxycrylène, l'octocrylène et le benzoate de phényle. Le diacétylresvératryl thioctate est solubilisé d'une manière stable un jour après (1J) la mise en solution.

Il est à noter que tous ces solvants remplissent les conditions $\delta_h \leq 8$ et $\delta_d \geq 6,9$ à température ambiante, tandis que l'octyldodécanol et le PPG-15 éther stéarylique ont les valeurs de $\delta_h$ et de $\delta_d$ en dehors de ces gammes.

Parmi les cinq solvants ayant démontré des résultats positifs, l'éthylhexyle méthoxycrylène et l'octocrylène sont particulièrement satisfaisants.

Il est à noter que ces deux solvants remplissent, en plus des deux conditions $\delta_h \leq 8$ et $\delta_d \geq 6,9$ à température ambiante, les conditions supplémentaires suivantes : $4,7 \leq \delta_p \leq 7,5$, $18 \leq \delta_T \leq 22$ et $6 \leq \log(K_{ow}) \leq 7,5$ à température ambiante.

L'octocrylène a été choisi pour les exemples suivants.

**Exemple 1-b) :** Exemple comparatif

La stabilisation du resveratrol par l'isosorbide de diméthyle, l'éthylhexyle méthoxycrylène et l'octocrylène a été évaluée selon le même protocole que l'Exemple 1-a), sauf que la solubilité a été évaluée le même jour que $t_0$ (0J).

**Résultats**

Le tableau de la page suivante présente les valeurs des paramètres de solubilisation de chaque solvant testé ($\delta_h$, $\delta_d$, $\delta_p$, $\delta_T$ et $\log(K_{ow})$ à température ambiante) et les résultats des tests de stabilisation du resveratrol.

En conclusion, l'actif resvératrol précipite pour les trois solvants testés.

| | Paramètres de solubilisation | | | | | solubilité | | Stabilité |
|---|---|---|---|---|---|---|---|---|
| | $\delta_h$ | $\delta_d$ | $\delta_p$ | $\delta_T$ | Log ($K_{ow}$) | $t_0/T_a$ | 0J/$T_a$ | |
| Isosorbide de diméthyle | 7,6 | 17,5 | 7,4 | 20 | -0,43 | Partiellement soluble | Précipité | non |
| Ethylhexyle méthoxycrylène | 6,4 | 18, | 6,8 | 20,2 | 6,99 | fraction soluble | Précipité | non |
| Octocrylène | 5,6 | 18,2 | 6,6 | 19,5 | 6,96 | fraction soluble | Précipité | non |

**Exemple 2 : Choix d'absorbeur d'odeur**

Des absorbeurs d'odeur ont été sélectionnés selon le protocole suivant.

**Protocole**

La composition de la Base suivante a été préparée. Dans cette composition, l'octocrylène est utilisé comme solvant stabilisateur du diacétylresvératryl thioctate.

| Eau déminéralisée | 85,75 % |
| Gomme de guar | 0,30 % |
| Sodium acrylate / acryloyldimethyl taurate dimethylacrylamide crosspolymer & isohexadecane & Polysorbate 60 (Simulgel SMS 88) | 1,50 % |
| Glycérine | 2,00 % |
| Disodium EDTA (Dissolvine NA-2) | 0,05 % |
| Phénoxyéthanol | 0,70 % |
| Diacétylresvératryl thioctate | 0,10 % |
| Trioctyl Trimellitate (Biosynth Totm) | 3, 00 % |
| Dimyristyl tartrate / Cetearyl alcohol - C12-15 Pareth 7 - PPG 25 Laureth 25 (Cosmacol PSE) | 2,00 % |
| Octocrylène | 4,00 % |

0,60 % de chaque absorbeur d'odeur candidat a été ajoutés à la base ci-dessus.

Premièrement, l'odeur initiale ($t_0$) a été évaluée à température ambiante ($T_a$) .

Pour les solvants dont les résultats de cette première évaluation ont été positifs, une deuxième évaluation olfactive a été effectuée 15 jours après à 45°C (15J/45°C).

**Résultats**

Les résultats sont résumés dans le tableau ci-dessous.

| Absorbeur d'odeur | $t_0/T_a$ | 15J/45 °C |
|---|---|---|
| Magnésium aluminium silicate hydraté | oui | oui |
| Oxide de zinc | oui | oui |
| Kaolin | oui | oui |
| Poudre de cuivre et silice | oui | oui |
| $Cu_2CO_3(OH)_2$ (ou malachite) | oui | oui |
| Silice | non | - |
| Alminium hydraté | non | - |
| Gluconate de zinc | non | - |
| $Cu_2CO_3(OH)_2$ (ou malachite) + EDTA | non | - |
| Polyméthacrylate de méthyle | non | - |
| Bis-éthylhexyle hydroxydiméthoxy benzylmalonate | non | - |
| Sel de zinc et de L-pyrrolidone carboxylate | non | - |

Après la première évaluation olfactive à température ambiante à $t_0$ ($t_0/T_a$), le magnésium aluminium silicate hydraté, l'oxyde de zinc, le kaolin, la poudre de cuivre et silice, la $Cu_2CO_3(OH)_2$ (ou malachite) ont été retenus. Après la deuxième évaluation 15 jours après à 45°C (15J/45°C), des résultats satisfaisants ont été obtenus pour ces composés.

Exemple **3 :** Etude de la stabilité olfactive

Pour les absorbeurs d'odeur retenus dans l'exemple 2 (le magnésium aluminium silicate hydraté, l'oxyde de zinc, le kaolin, la poudre de cuivre et silice, le sel de cuivre et de L-pyrrolidone carboxylate et la $Cu_2CO_3(OH)_2$ (ou malachite)) ainsi que pour le sel de cuivre et de L-pyrrolidone carboxylate, une étude de la stabilité olfactive plus détaillée a été menée selon le protocole suivant.

**Protocole**

Une composition de Base suivante a été préparée. Dans cette composition, l'octocrylène est utilisé comme solvant stabilisateur du diacétylresvératryl thioctate.

| Base | |
|---|---|
| Eau déminéralisée % | 89,80 |
| (90,34 % pour le sel de cuivre et de L-pyrrolidone carboxylate) | |
| Gomme de guar | 0,30 % |
| Sodium acrylate / acryloyldimethyl taurate dimethylacrylamide crosspolymer & isohexadecane & Polysorbate 60 (Simulgel SMS 88) | 1,50 % |
| Glycérine | 2,00 % |
| Phénoxyéthanol | 0,70 % |
| Dimyristyl tartrate / Cetearyl alcohol - C12-15 Pareth 7 - PPG 25 Laureth 25 (Cosmacol PSE) | 1,00 % |
| Diacétylresvératryl thioctate | 0,10 % |
| Octocrylène | 4,00 % |

0,6 % de chaque absorbeur d'odeur candidat a été ajouté à l'une des compositions de base ci-dessus et la stabilité olfactive a été testée à trois températures (température ambiante, 5°C et 45°C) et aux temps suivants :

- au début ($t_0$),

- 1 mois (1M),

- 2 mois (2M) et

- 3 mois (3M).

Les tableaux ci-dessous présentent les résultats. Les résultats à $t_0$, 1 mois (1M) et 3 mois (3M) sont présentés en une seule colonne pour les trois températures testées, car les résultats sont identiques.

**Résultats**

Les résultats sont résumés dans le tableau ci-dessous.

| Base | $t_0$ | 1M | 2M | | | 3M |
|---|---|---|---|---|---|---|
| | | | $T_a$ | 5°C | 45°C | |
| Référence (Base) | oui | oui | non | non | non | non |
| Magnésium aluminium silicate hydraté | oui | non | non | non | non | non |
| Oxide de zinc | oui | oui | oui | oui | non | non |
| Kaolin | oui | oui | oui | oui | oui | oui |
| $Cu_2CO_3(OH)_2$ (ou malachite) | oui | oui | oui | oui | oui | oui |
| Sel de cuivre et de L-pyrrolidone carboxylate | oui | oui | oui | oui | oui | oui |

Les résultats montrent que le mélange de Base avec le magnésium aluminium Silicate hydraté perd son efficacité à mois (1M).

De même, au-delà de deux mois (2M et 3M), les mélanges de Base avec l'oxyde de zinc ne sont plus efficaces.

Au final, le kaolin et la $Cu_2CO_3(OH)_2$ (ou malachite) donnent des résultats satisfaisants. Le sel de cuivre et de L-pyrrolidone carboxylate est le plus approprié en combinaison avec la Base.

**Exemple 4 : Stabilité du diacétylresvératryl thioctate dans la formulation**

Un test de stabilité du diacétylresvératryl thioctate a été effectué pour les absorbeurs d'odeurs retenus dans l'Exemple 3, selon le protocole suivant, dans le but de confirmer la compatibilité entre le solvant et l'absorbeur d'odeur.

**Protocole**

0,6 % de chaque absorbeur d'odeur candidat a été ajouté à la Base et la stabilité olfactive a été testée à deux températures (5°C et 45°C) à 2 mois.

**Résultats**

Les résultats sont présentés dans les tableaux ci-dessous.

| Base | 5°C | 45°C |
|---|---|---|
| Contrôle sans absorbeur d'odeur (Base) | 0.098 | 0.095 |
| Magnésium aluminium silicate hydraté | 0,096 | 0,094 |
| Oxyde de zinc | 0,094 | 0,064 |
| Kaolin | 0,095 | 0,09 |
| $Cu_2CO_3(OH)_2$ 2 (ou malachite) | 0,097 | 0,085 |
| Sel de cuivre et de L-pyrrolidone carboxylate | 0,1 | 0,094 |

A 5°C, des résultats satisfaisants ont été obtenus pour tous les absorbeurs testés, tandis qu'à 45°C, l'oxide de zinc perturbe la stabilisation du diacétylresvératryl thioctate.

Un taux de stabilisation du diacétylresvératryl thioctate supérieur ou égal à 0.085 a été obtenu pour le magnésium aluminium silicate hydraté, le kaolin, la $Cu_2CO_3(OH)_2$ (ou malachite) et le sel de cuivre et de L-pyrrolidone carboxylate.

**Exemple 5 : Formule selon l'invention**

La composition suivante est une émulsion eau dans huile et a été préparée pour prévenir et/ou lutter contre le vieillissement cutané.

| | |
|---|---|
| Eau | Qsp 100 |
| Alcool | 10 % |
| Glycérine | 2 % |
| Phénoxyéthanol | qs |
| Octyldodécanol | 4 % |
| Acryloyldiméthyltaurate d'ammonium / VPcopolymère (ARISTOFLEX AVC) | 1 % |
| Kaolin (WHITETEX) | 0,6 % |
| Sel de cuivre et de L-pyrrolidonecarboxylate) (CUIVRIDONE) | 0,06 % |
| Octocrylène | 3 % |
| Diacétylresvératryl thioctate (reversage) | 0.1 % |
| Hétérosides de centella | 3 % |

**Revendications**

1. Composition cosmétique comprenant:

   - le diacétylresvératryl thioctate; et
   - un solvant ;

   **caractérisée en ce que** ledit solvant a des paramètres de solubilité de Hansen de $\delta_h$ inférieure ou égale à 8 et de $\delta_d$ supérieure ou égale à 16,9 à température ambiante.

2. Composition cosmétique selon la revendication 1, **caractérisée en ce que** ledit solvant a une valeur de $\delta_p$ comprise entre 4,7 et 7,5 à température ambiante.

**3.** Composition cosmétique selon la revendication 1 ou 2, **caractérisée en ce que** ledit solvant a une valeur de $\delta_T$ comprise entre 18 et 22 à température ambiante.

**4.** Composition cosmétique selon l'une quelconque des revendications précédentes, **caractérisée en ce que** ledit solvant a une valeur de $\log(K_{ow})$ comprise entre 6 et 7,5 à température ambiante.

**5.** Composition cosmétique selon l'une quelconque des revendications précédentes, **caractérisée en ce que** ledit solvant est choisi parmi : trioctyl trimellitate, isosorbide de diméthyle, éthylhexyle méthoxycrylène, octocrylène, benzoate de phényle et leurs mélanges.

**6.** Composition cosmétique selon l'une quelconque des revendications précédentes, **caractérisée en ce qu'**elle comprend en outre un absorbeur d'odeur choisi parmi : magnésium aluminium silicate hydraté, oxyde de zinc, kaolin, $Cu_2CO_3(OH)_2$ (ou malachite), sel de cuivre et de L-pyrrolidone carboxylate et leurs mélanges.

**7.** Composition cosmétique selon l'une quelconque des revendications précédentes, **caractérisée en ce qu'**elle comprend le diacétylresvératryl thioctate en une proportion allant de 0,001% à 15% en poids, et de préférence de 0,01% à 10% en poids par rapport au poids total de la composition.

**8.** Composition cosmétique selon l'une quelconque des revendications précédentes, **caractérisée en ce qu'**elle comprend le solvant en une proportion allant de 0,1% à 50% en poids, et de préférence de 1% à 25% en poids par rapport au poids total de la composition.

**9.** Composition cosmétique selon l'une quelconque des revendications précédentes, **caractérisée en ce qu'**elle comprend l'absorbeur d'odeur en une proportion allant de 0,0006 % à 6 % en poids, et de préférence de 0,006 % à 0,6 % en poids par rapport au poids total de la composition.

**10.** Utilisation de la composition cosmétique selon l'une quelconque des revendications précédentes, pour prévenir et/ou lutter contre le vieillissement cutané.

**Patentansprüche**

**1.** Kosmetische Zusammensetzung, welche umfasst:

- Diacetylresveratrylthioctat; sowie
- ein Lösungsmittel;

**dadurch gekennzeichnet, dass** das Lösungsmittel bei Raumtemperatur Hansen-Löslichkeitsparameter von $\delta_h$ kleiner oder gleich 8 und $\delta_d$ größer oder gleich 16,9 aufweist.

**2.** Kosmetische Zusammensetzung nach Anspruch 1, **dadurch gekennzeichnet, dass** das Lösungsmittel bei Raumtemperatur einen Wert $\delta_p$ zwischen 4,7 und 7,5 aufweist.

**3.** Kosmetische Zusammensetzung nach Anspruch 1 oder 2, **dadurch gekennzeichnet, dass** das Lösungsmittel bei Raumtemperatur einen Wert $\delta_T$ zwischen 18 und 22 aufweist.

**4.** Kosmetische Zusammensetzung nach einem der vorhergehenden Ansprüche, **dadurch gekennzeichnet, dass** das Lösungsmittel bei Raumtemperatur einen Wert $\log(K_{ow})$ zwischen 6 und 7,5 aufweist.

**5.** Kosmetische Zusammensetzung nach einem der vorhergehenden Ansprüche, **dadurch gekennzeichnet, dass** das Lösungsmittel gewählt ist aus: Trioctyltrimellitat, Dimethylisosorbid, Ethylhexyl-Methoxycrylen, Octocrylen, Phenylbenzoat und deren Mischungen.

**6.** Kosmetische Zusammensetzung nach einem der vorhergehenden Ansprüche, **dadurch gekennzeichnet, dass** sie ferner einen Geruchsabsorber umfasst, welcher gewählt ist aus: hydriertem Magnesium-Aluminium Silikat, Zinkoxyd, Kaolin, $Cu_2CO_3(OH)_2$ (oder Malachit), Kupfersalz und L-Pyrrolidon-Carboxylat und deren Mischungen.

**7.** Kosmetische Zusammensetzung nach einem der vorhergehenden Ansprüche, **dadurch gekennzeichnet, dass** sie Diacetylresveratrylthioctat mit einem Anteil von 0,001 bis 15 Gewichtsprozent und vorzugsweise von 0,01 bis 10 Gewichtsprozent am Gesamtgewicht der Zusammensetzung aufweist.

8.  Kosmetische Zusammensetzung nach einem der vorhergehenden Ansprüche, **dadurch gekennzeichnet, dass** sie das Lösungsmittel mit einem Anteil von 0,1 bis 50 Gewichtsprozent und vorzugsweise von 1 bis 25 Gewichtsprozent am Gesamtgewicht der Zusammensetzung aufweist.

9.  Kosmetische Zusammensetzung nach einem der vorhergehenden Ansprüche, **dadurch gekennzeichnet, dass** sie den Geruchsabsorber mit einem Anteil von 0,0006 bis 6 Gewichtsprozent und vorzugsweise von 0,006 bis 0,6 Gewichtsprozent am Gesamtgewicht der Zusammensetzung aufweist.

10. Verwendung der kosmetischen Zusammensetzung nach einem der vorhergehenden Ansprüche zur Prävention und/oder Hemmung der Hautalterung.

**Claims**

1.  A cosmetic composition comprising:

    - diacetylresveratryl thioctate; and
    - a solvent;

    **characterized in that** said solvent has Hansen solubility parameters: $\delta_h$ value less than or equal to 8 and $\delta_d$ greater than or equal to 16.9 at ambient temperature.

2.  The cosmetic composition as claimed in claim 1, **characterized in that** said solvent has a $\delta_p$ value between 4.7 and 7.5 at ambient temperature.

3.  The cosmetic composition as claimed in claim 1 or 2, **characterized in that** said solvent has a $\delta_T$ value between 18 and 22 at ambient temperature.

4.  The cosmetic composition as claimed in any one of the preceding claims, **characterized in that** said solvent has a log ($K_{ow}$) value between 6 and 7.5 at ambient temperature.

5.  The cosmetic composition as claimed in any one of the preceding claims, **characterized in that** said solvent is chosen from: trioctyl trimellitate, dimethyl isosorbide, ethylhexyl methoxycrylene, octocrylene, phenyl benzoate and mixtures thereof.

6.  The cosmetic composition as claimed in any one of the preceding claims, **characterized in that** it also comprises an odour absorber chosen from: magnesium aluminium silicate hydrate, zinc oxide, kaolin, $Cu_2CO_3(OH)_2$ (or malachite), copper L-pyrrolidone carboxylate salt, and mixtures thereof.

7.  The cosmetic composition as claimed in any one of the preceding claims, **characterized in that** it comprises the diacetylresveratryl thioctate in a proportion ranging from 0.001% to 15% by weight, and preferably from 0.01% to 10% by weight, relative to the total weight of the composition.

8.  The cosmetic composition as claimed in any one of the preceding claims, **characterized in that** it comprises the solvent in a proportion ranging from 0.1% to 50% by weight, and preferably from 1% to 25% by weight, relative to the total weight of the composition.

9.  The cosmetic composition as claimed in any one of the preceding claims, **characterized in that** it comprises the odour absorber in a proportion ranging from 0.0006% to 6% by weight, and preferably from 0.006% to 0.6% by weight, relative to the total weight of the composition.

10. The use of the cosmetic composition as claimed in any one of the preceding claims, for preventing and/or combating skin aging.

## RÉFÉRENCES CITÉES DANS LA DESCRIPTION

**Documents brevets cités dans la description**

- WO 2006134282 A

- WO 2009010356 A

**Littérature non-brevet citée dans la description**

- Dictionnaire CTFA (International Cosmetic Ingrédient Dictionary and Handbook. The Cosmetic, Toiletry and Fragrance Association, 2006